# EUROPÄISCHE PATENTSCHRIFT

(11) **EP 2 334 236 B1**
(45) Veröffentlichungstag und Bekanntmachung des Hinweises auf die Patenterteilung: **24.04.2019**
(21) Anmeldenummer: 09783689.4
(22) Anmeldetag: 02.10.2009
(51) Int. Cl.: A61B 5/1459

(54) **IMPLANTIERBARES SENSORELEMENT**
IMPLANTABLE SENSOR ELEMENT
ELÉMENT DE CAPTEUR IMPLANTABLE

(30) Priorität: 02.10.2008 EP 08165703
(43) Veröffentlichungstag der Anmeldung: 22.06.2011
(73) Patentinhaber: EyeSense AG, 4051 Basel (CH)
(72) Erfinder: MÜLLER, Achim, 63762 Grossostheim (DE); HERBRECHTSMEIER, Peter, 61462 Königstein (DE)
(74) Vertreter: Herzog, Fiesser & Partner Patentanwälte PartG mbB
(86) Internationale Anmeldenummer: PCT/EP2009/062823
(87) Internationale Veröffentlichungsnummer: WO 2010/037847

(56) Entgegenhaltungen:
- EP-A- 0 340 105
- EP-A- 0 589 862
- EP-A- 1 810 665
- WO-A-02/30275
- US-A- 4 582 809
- US-A- 4 842 783
- US-A- 5 353 792
- US-A1- 2006 024 357
- US-B1- 6 256 522

## Beschreibung

Die Erfindung betrifft ein Sensorelement zur Erfassung mindestens eines Analyten in einer Körperflüssigkeit oder einem Körpergewebe. Die Erfindung betrifft weiterhin eine Sensoranordnung, welche ein erfindungsgemäßes Sensorelement und einen optischen Detektor umfasst. Weiterhin betrifft die Erfindung ein Verfahren zum Erzeugen eines Sensorelements. Derartige Sensorelemente, Sensoranordnungen und Verfahren werden insbesondere für die Bestimmung mindestens einer Metabolitkonzentration in einer Körperflüssigkeit und/oder einem Körpergewebe eingesetzt. Derartige Metaboliten können beispielsweise, jedoch nicht ausschließlich, Blutglucose, Lactat, Cholesterin oder andere Arten von Analyten bzw. Metaboliten umfassen. Alternativ oder zusätzlich lässt sich das Sensorelement bzw. die Sensoranordnung jedoch auch in anderen Bereichen der Analytik einsetzen, beispielsweise im Bereich der chemischen Analytik, insbesondere der in-situ-Analytik, der Prozessüberwachung oder in ähnlichen Bereichen.

Herkömmliche Systeme zur Bestimmung von Analyt- bzw. Metabolitkonzentrationen basieren in vielen Fällen auf der Generierung einer Probe einer Körperflüssigkeit, wie beispielsweise eines Blutstropfens, welche anschließend mittels eines geeigneten Messgerätes auf ihren Analytgehalt untersucht wird. Hierbei können beispielsweise optische und/oder elektrochemische Messverfahren zum Einsatz kommen.

Um die mit der häufigen Generierung von Blutproben verbundenen Unannehmlichkeiten der Patienten zu verringern, wurden verschiedene nicht-invasive oder minimal-invasive Technologien zur Messung von Analytkonzentrationen entwickelt. Im Folgenden wird dabei ohne Beschränkung des Schutzumfangs der Erfindung auf die Bestimmung der Blutglucosekonzentration eingegangen, wobei jedoch naturgemäß alternativ oder zusätzlich auch andere Arten von Analyten bzw. Metaboliten nachweisbar sind.

Die invasiven Technologien zur Bestimmung der Analytkonzentration basieren üblicherweise auf in eine Körpergewebe und/oder eine Körperflüssigkeit implantierbaren Sensoren, welche auf optische und/oder elektrochemische Weise die Analytkonzentration bestimmen können.

Optische Systeme verwenden dabei in der Regel mindestens ein Sensormaterial, welches bei Anwesenheit eines oder mehrerer bestimmter Analyten mindestens eine optisch messbare Eigenschaft ändert. Diese optisch messbare Eigenschaft kann auf verschiedenste Weisen ausgestaltet sein, wobei viele verschiedene Verfahren, Sensormaterialien und Messvorrichtungen aus dem Stand der Technik bekannt sind. Sämtliche dieser bekannten Sensormaterialien lassen sich grundsätzlich auch im Rahmen der vorliegenden Erfindung einsetzen.

So beschreibt beispielsweise WO 01/13783 einen Okularsensor für Glucose, welcher als Augenlinse ausgestaltet ist. Der Okularsensor umfasst als Sensormaterial einen Glucoserezeptor, welcher mit einem ersten Fluoreszenzlabel markiert ist, und einen Glucose-Konkurrenten, welcher mit einem zweiten Fluoreszenzlabel ("Donor") markiert ist. Die beiden Fluoreszenzlabel sind derart gewählt, dass, wenn der Konkurrent an den Rezeptor gebunden ist, die Fluoreszenz des zweiten Fluoreszenzlabels aufgrund eines resonanten Fluoreszenz-Energietransfers gelöscht wird (Quenching). Durch Überwachen der Veränderung der Fluoreszenzintensität bei einer Wellenlänge um das Fluoreszenzmaximum des quenchbaren Fluoreszenzlabels kann der Anteil des Fluoreszenz-markierten Konkurrenten gemessen werden, welcher durch die Glucose verdrängt worden ist. Auf diese Weise kann die Glucosekonzentration in der Augenflüssigkeit bestimmt werden. Die Messung kann wiederum genutzt werden, um daraus auf die Blutglucosekonzentration zu schließen. Auch andere Arten von Nachweisen sind denkbar und dem Fachmann geläufig, beispielsweise ein Fluoreszenznachweis des ersten Fluoreszenzlabels.

WO 02/087429 beschreibt ein Fluoreszenz-Photometer, mittels dessen Blutglucosekonzentrationen durch Messung der Glucosekonzentrationen an der Augenflüssigkeit bestimmt werden können. Die dargestellte Vorrichtung ist in der Lage, gleichzeitig zwei Fluoreszenzintensitäten bei verschiedenen Wellenlängen zu messen.

Eine Herausforderung bei der Verwendung optischer Nachweissysteme mittels eines optischen Sensormaterials in implantierten Sensoren besteht jedoch naturgemäß stets darin, optische Signale von einem Messgerät hin zum Sensormaterial und/oder in umgekehrter Richtung, d. h. vom Sensormaterial zum Messgerät, zu leiten. Bei den in WO 01/13783 und in WO 02/087429 beschriebenen Vorrichtungen spielt diese Problematik eine untergeordnete Rolle, da die Gewebeschichten, welche den implantierten Sensor bedecken, in der Regel im Augenbereich transparent sind und somit ein Ein- bzw. Auskoppeln von Lichtsignalen ermöglichen. Die technische Herausforderung der optischen Kopplung wächst jedoch, wenn Sensoren in nicht-transparente Hautpartien implantiert werden. Die vorliegende Erfindung ist somit nicht auf die Verwendung im Augenbereich beschränkt, sondern beinhaltet auch die Möglichkeit einer Implantation in Körperbereichen, in welchen der implantierte Sensor durch nicht-transparente Gewebepartien bedeckt ist.

Zur Umgehung der beschriebenen Problematik der optischen Kopplung sind aus dem Stand der Technik verschiedene Systeme bekannt. So beschreibt beispielsweise WO 2005/054831 A1 ein Sensorelement zur Bestimmung einer Glucosekonzentration, bei welchem ein optischer Lichtwellenleiter verwendet wird. Am distalen Ende des Lichtwellenleiters wird ein Sensorelement auf denselben aufgebracht, welches ein bindendes Protein umfasst, das mit mindestens einem Ziel-Analyt binden kann. Das Sensorelement umfasst weiterhin mindestens ein Reporter-Gruppe, welche einer Lumineszenzänderung unterliegt, wenn sich die Analytkonzentrationen ändern. Optional umfasst das Sensorelement Referenzgruppen mit Lumineszenzeigenschaften, die sich im Wesentlichen nicht ändern, wenn sich die Analytkonzentrationen ändern.

In D. Meadows and J. S. Schultz: Fiber-optic biosensors based on fluorescence energy transfer, Talanta, Vol. 35, No. 2, pp. 145-150, 1988, wird eine biochemische Methode zum Test auf Glucose beschrieben, welche auf einem Fluoreszenz-Energietransfer beruht. Unter anderem wird dabei vorgeschlagen, optische Lichtwellenleiter einzusetzen, um an ein Sensorelement anzukoppeln. Das Sensorelement umfasst eine hohle Dialysefaser, durch welche der nachzuweisende Analyt, in diesem Fall Glucose, hindurchdiffundieren kann, um zu dem im Inneren der Dialysefaser befindlichen Sensormaterial zu gelangen.

US 7,226,414 B2 beschreibt eine Glucose-Sensorvorrichtung zur Implantation innerhalb des subkutanen Gewebes eines tierischen Körpers. Ein Sensormaterial ist in einer ersten Kammer angeordnet, wobei Glucose aus dem Körpergewebe in die erste Kammer eintreten kann. Das Sensorelement umfasst weiterhin eine Referenzkammer mit einer Referenzlösung. Zum Ankoppeln eines Auslesegerätes wird wieder die Verwendung von Lichtwellenleiter-Fasern vorgeschlagen, welche ein Nachweisgerät mit den Kammern verbinden.

In US 2007/0122829 A1 werden ein System, eine Vorrichtung und ein Verfahren zur Messung der Konzentration eines Analyten in einer Flüssigkeit oder einer Matrix vorgeschlagen. Ein thermodynamisch stabilisierter, Analyt-bindender Ligand wird vorgeschlagen. Auch in diesem Fall wird wiederum die Verwendung eines an ein Sensorelement angekoppelten separaten Lichtwellenleiters in Form einer Faser vorgeschlagen, welche ein Nachweisgerät mit einem implantierten Sensorelement verbindet.

Abgesehen von den offenbarten Sensormaterialien, welche beispielsweise auch im Rahmen der vorliegenden Erfindung einsetzbar sind, weisen die WO 2005/054831 A1, die Veröffentlichung von D. Meadows et al., die US 7,226,414 B2 und die US 2007/0122829 A1 jedoch in der Praxis erhebliche Nachteile auf. So besteht ein erheblicher Nachteil insbesondere in der aufwendigen Herstellung derartiger Sensorelemente, da zunächst das eigentliche Sensorelement hergestellt werden muss, dieses dann mit einer entsprechenden Lichtwellenleiter-Faser verbunden werden muss, um anschließend diese Anordnung zu implantieren. Da Lichtwellenleiter-Fasern in der Praxis eine erhebliche Empfindlichkeit gegenüber mechanischen Belastungen aufweisen, kann es zudem vorkommen, dass bei der Implantation die Lichtwellenleiter beschädigt werden, wodurch die Funktionalität der Sensorelemente beeinträchtigt oder gar verhindert wird. Weiterhin sind für das Entfernen der Sensorelemente einschließlich der optischen Fasern teilweise erhebliche Eingriffe in das Körpergewebe erforderlich, da ein Herausziehen der Lichtwellenleiter-Faser aus dem Körpergewebe in der Regel eine Ablösung des Sensorelements von der Lichtwellenleiter-Faser b

US 5,353,792 und WO 02/30275 A1 offenbaren ein Sensorelement zur Erfassung mindestens eines Analyten in einer Körperflüssigkeit oder einem Körpergewebe wobei das Sensorelement einen implantierbaren, einstückigen Formkörper umfasst, wobei der Formkörper ein Sensorende und ein Koppelende umfasst.

Aufgabe der vorliegenden Erfindung ist es daher, ein Sensorelement sowie ein Verfahren zu dessen Herstellung bereitzustellen, welche die oben beschriebenen Nachteile bekannter Sensorelemente und Verfahren vermeiden. Insbesondere soll das Sensorelement eine zuverlässige und mechanisch stabile Ankopplung eines optischen Detektors ermöglichen, wobei gleichzeitig eine möglichst schmerzarme und schnelle Implantation gewährleistet sein soll.

Diese Aufgabe wird durch die Erfindung mit den Merkmalen der unabhängigen Ansprüche gelöst. Vorteilhafte Weiterbildungen der Erfindung, welche einzeln oder auch in Kombination verwirklicht sein können, sind in den Unteransprüchen gekennzeichnet.

Es wird ein Sensorelement zur Erfassung mindestens eines Analyten in einer Körperflüssigkeit oder einem Körpergewebe vorgeschlagen, welches insbesondere zur Bestimmung mindestens einer Metabolitkonzentration in einer Körperflüssigkeit eingesetzt werden kann. Als mögliche Beispiele von Analyten kann auf die obige Beschreibung des Standes der Technik verwiesen werden. Unter dem Begriff "Erfassung" kann dabei eine quantitative und/oder eine qualitative Bestimmung einer Analytkonzentration verstanden werden, d.h. eine Bestimmung der Menge und/oder Konzentration des Analyten in der Körperflüssigkeit und/oder die Beantwortung der Frage, ob der Analyt überhaupt in der Körperflüssigkeit enthalten ist.

Das Sensorelement umfasst einen implantierbaren, einstückigen Formkörper. Unter "implantierbar" ist dabei zu verstehen, dass der Formkörper im Wesentlichen aus biokompatiblen Materialien hergestellt ist und/oder eine biokompatible Beschichtung aufweist, so dass dieser Formkörper über längere Zeit hinweg (beispielsweise über mehrere Tage und/oder Wochen) im implantierten Zustand in einem Körpergewebe verbleiben kann, ohne Abstoßungsreaktionen und/oder Entzündungen und/oder Vergiftungen des Körpergewebes hervorzurufen. Unter "einstückig" ist dabei zu verstehen, dass der Formkörper im Wesentlichen als ein einzelnes Formteil ausgebildet ist, welches auch unter mechanischer Belastung (beispielsweise üblichen, beim Implantieren oder Entfernen des Sensorelements aus dem Körpergewebe auftretenden Druck- und/oder Zugbelastungen, sich nicht in mehrere Teile zerlegt. Insbesondere kann der Begriff "einstückig" auch beinhalten, dass das Formteil in einem einzelnen Arbeitsschritt hergestellt werden kann. Insbesondere sollen, wie unten beschrieben, ein Sensorbereich und ein Koppelteil sich bei derartigen Belastungen nicht voneinander lösen.

Der Formkörper kann insbesondere von insgesamt länglicher Gestalt sein, d. h. von einer Gestalt mit einer Länge, welche größer ist als der Durchmesser des Formkörpers. Beispielsweise kann der Formkörper eine zylinderförmige Gestalt mit rundem oder polygonalem Querschnitt aufweisen. Insgesamt sollte der Formkörper eine Länge aufweisen, welche zumindest näherungsweise der summierten Dicke von Dermis und Epidermis entspricht, so dass bei implantiertem Sensorelement das Sensorende in den unteren Dermis-Schichten oder in den Schichten der Unterhaut (Subcutis) angeordnet ist, um dort den Analyten zu detektieren.

Der Sensorbereich umfasst mindestens ein Sensormaterial, welches bei Anwesenheit des Analyten mindestens eine optisch messbare Eigenschaft ändert. Das Sensormaterial ist dabei derart ausgestaltet, dass dieser Sensor sensitiv auf den mindestens einen nachzuweisenden Analyten reagiert. Vorzugsweise ist diese Sensoreigenschaft spezifisch für den nachzuweisenden Analyten. Dabei können, wie aus dem oben beschriebenen Stand der Technik bekannt, verschiedene Nachweisprinzipien eingesetzt werden. Beispielsweise kann der Analyt mit dem Sensormaterial chemisch reagieren (zum Beispiel eine kovalente Bindung, eine komplexe Bindung oder eine ähnliche Verbindung eingehen), wobei diese Bindung beispielsweise durch Änderung von Fluoreszenzeigenschaften und/oder Farbeigenschaften des Analyten und/oder des Sensormaterials und/oder der Sensormaterial-Analyt-Verbindung nachgewiesen werden kann. Auch lockerere Bindungen sind möglich, beispielsweise physikalische Bindungen und/oder Annäherungen von Sensormaterial und Analyt, welche beispielsweise wiederum spektroskopisch nachgewiesen werden können. In jedem Fall ist das Sensormaterial jedoch derart ausgestaltet, dass sich mindestens eine optisch nachweisbare physikalische und/oder chemische Eigenschaft des Implantats ändert, wenn sich die Analytkonzentration in der Umgebung des Sensorelements ändert bzw. wenn in der Umgebung des Sensorelements ein Analyt vorhanden ist.

Zur Ankopplung bzw. Auskopplung von Licht aus dem Sensorbereich weist der Formkörper weiterhin mindestens ein optisch transparentes Koppelteil auf. Dieses Koppelteil, welches von dem Sensorbereich räumlich getrennt, jedoch einstückig mit diesem zu einem Formteil ausgebildet ist, ist eingerichtet, um elektromagnetische Strahlung in mindestens einem Spektralbereich zwischen dem Sensorbereich und dem Koppelende zu übertragen, d. h. zu transmittieren. Im Gegensatz zu den aus dem Stand der Technik bekannten Lichtwellenleiter-Fasern, mittels derer an die implantierten Sensorelemente angekoppelt wird, wird also im Rahmen der vorliegenden Erfindung vorgeschlagen, das Koppelteil einstückig mit dem Sensorbereich zu verbinden. Hierdurch wird eine erhöhte mechanische Stabilität gewährleistet, da insbesondere an der Verbindungsstelle zwischen Koppelteil und Sensorbereich nunmehr keine unter Zugbelastungen auflösbaren Verbindungen mehr bestehen, welche bei einer Implantation oder einer Entfernung des Sensorelements aus einem Gewebe zu einer Zerstörung des Sensorelements führen könnten.

Des Weiteren muss das Koppelteil nicht notwendigerweise als "Lichtwellenleiter" im klassischen Sinne ausgebildet sein, sondern kann beispielsweise lediglich ein "Fenster" darstellen, mittels dessen optisch an den Sensorbereich angekoppelt werden kann. Im implantierten Zustand kann insbesondere das Koppelende des Koppelteils noch unterhalb der obersten Hautschicht angeordnet sein, so dass eine optische Kopplung durch diese oberste Hautschicht, welche noch weitgehend transparent ist (zumindest im sichtbaren, infraroten und/oder ultravioletten Spektralbereich) erfolgen kann. Auf Lichtwellenleiter-Eigenschaften, wie insbesondere Eigenschaften von Lichtwellenleitern mit Kern und Hülle zur Anpassung des Brechnungsindex oder Gradientenfasern kann weitgehend verzichtet werden.

Diese Einstückigkeit zwischen Formkörper und Sensorbereich wird erfindungsgemäß dadurch realisiert, dass der Formkörper im Sensorbereich mindestens ein optisch transparentes Matrixmaterial aufweist. Das Matrixmaterial ist derart gewählt, dass der mindestens eine nachzuweisende Analyt zumindest teilweise durch das Matrixmaterial zu dem in dem Matrixmaterial eingebetteten Sensormaterial diffundieren kann. Gleichzeitig ist jedoch auch das Koppelteil zumindest teilweise durch das Matrixmaterial gebildet. Hierdurch wird die oben beschriebene Einstückigkeit des Formkörpers gewährleistet, da sich der Sensorbereich und das Koppelteil nunmehr im Wesentlichen lediglich dadurch unterscheiden, dass im Sensorbereich das Sensormaterial eingebettet ist, wohingegen das Koppelteil im Wesentlichen frei von Sensormaterial ist.

Unter einem Matrixmaterial kann dabei ein einphasiges Material zu verstehen sein, also ein Material, welches sowohl makroskopisch als auch mikroskopisch homogene Eigenschaften aufweist, beispielsweise ein Material, welches ein einzelnes Grundelement in vernetzter Form aufweist. Alternativ kann das Matrixmaterial jedoch auch als mehrphasiges Material ausgestaltet sein, also als Material, welches beispielsweise makroskopisch im Wesentlichen homogen ausgestaltet ist, mikroskopisch jedoch mehrere Phasen aufweist. Als Beispiel eines derartiges mikroskopisch heterogenen, mehrphasigen Systems sind Silikon-Hydrogele zu nennen, bei denen die Hydrogel-Phase nur eine von mehreren, nebeneinander vorliegenden Phasen ist. Alternativ oder zusätzlich lassen sich jedoch auch andere Matrixmaterialien einsetzen, beispielsweise statistische Copolymere, also Copolymere, bei denen zwei oder mehrere verschiedene Grundelemente in statistischer Reihenfolge aufeinander folgen. Auch derartige statistische Copolymere bilden üblicherweise homogene Phasen.

Das Koppelteil und der Sensorbereich werden insbesondere durch zumindest weitgehend zeitgleiches Aushärten des Matrixmaterials und/oder eines Precursors des Matrixmaterials im Sensorbereich und im Bereich des Koppelteils hergestellt. Unter einem Precursor ist dabei ein beliebiges Ausgangs- oder Zwischenprodukt zu verstehen, welches allein oder unter Einbeziehung weiterer Stoffe durch chemische Reaktion oder auch durch einen Phasenübergang das Matrixmaterial bilden kann. Das Sensormaterial kann in dem Matrixmaterial insbesondere durch eine geeignete Einbettung und/oder eine chemische Bindung immobilisiert werden, um ein Diffundieren des Sensormaterials in umgebendes Körpergewebe zu vermeiden, zumindest während der üblichen Zeiten, während derer das Sensorelement in dem Körpergewebe implantiert ist. Insbesondere kann das Sensorelement zumindest teilweise in Mikro- oder Nanopartikeln eingebettet sein, insbesondere in Mikro- oder Nanokapseln.

Weiterhin ist es bevorzugt, wenn das Matrixmaterial in dem Sensorbereich im implantierten Zustand des Sensorelements unmittelbar mit der Körperflüssigkeit und/oder dem Körpergewebe in Kontakt steht. Zu diesem Zweck kann insbesondere auf eine Ummantelung des Matrixmaterials im Bereich des Sensorelements verzichtet werden. Weiterhin kann auf aufwendige Membranen, wie beispielsweise die aus dem Stand der Technik bekannten Dialysemembranen verzichtet werden.

Wie oben beschrieben, kann das Koppelteil insbesondere als langgestrecktes Koppelteil mit einem im Wesentlichen homogenen Brechungsindex ausgebildet sein. Unter "langgestreckt" kann dabei ein Verhältnis zwischen Länge und Durchmesser verstanden werden, welcher mindestens bei 2, vorzugsweise bei 5 und besonders bevorzugt bei einem Faktor von ca. 10 oder mehr liegt. Der Durchmesser kann beispielsweise im Bereich zwischen 100 Mikrometern und 1 Millimeter liegen, insbesondere im Bereich zwischen 200 Mikrometern und 500 Mikrometern. Die Länge kann beispielsweise im Bereich zwischen einem und 8 Millimetern liegen, vorzugsweise im Bereich zwischen 2 und 5 Millimetern. Das Verhältnis zwischen Durchmesser und Länge liegt vorzugsweise im Bereich zwischen 1:5 und 1:20, insbesondere bei ca. 1:10. Die exakten Abmessungen und Dimensionen des Sensorelements können insbesondere auch an die Implantationsstelle des Sensorelements angepasst werden.

Das Matrixmaterial umfasst mindestens einen vernetzbaren Kunststoff, insbesondere einen biokompatiblen vernetzbaren Kunststoff, in vernetzter Form. Der vernetzbare Kunststoff kann vorzugsweise ein Hydrogel umfassen, da dieses Material seine guten Verarbeitungsqualitäten und seine hervorragende Biokompatibilität insbesondere im Bereich der Augenimplantate bereits mehrfach unter Beweis gestellt hat. Alternativ oder zusätzlich können jedoch auch beispielsweise ein Polymethylmethacrylat und/oder ein Polycarbonat und/oder ein Polystyrol und/oder ein Silicon oder Kombinationen der genannten und/oder anderer Materialien vorteilhaft eingesetzt werden.

Der vernetzbare Kunststoff kann insbesondere hergestellt sein unter Verwendung mindestens eines eines Nelfilcon-Polymers hergestellt sein. Derartige Polymere sind beispielsweise in EP 807 265 B1 oder in EP 790 258 B1 dargestellt. Es handelt sich dabei um vernetzbare bzw. vernetzte Polyvinylacetate oder ein vernetzbare Derivate des Polyvinylacetats, vernetzbarere Polyvinylalkohole oder ein vernetzbare Derivate des Polyvinylalkohols. Weiterhin können auch vernetzbare Polymere auf Polyethylenglycol-Basis verwendet werden, insbesondere auf Basis mindestens eines der folgenden Polymere: Bis Acrylol-Polyethylenglycol, Bis Acrylamido-Polyethylenglycol, ein Polyurethan auf der Basis von Polyethylenglycol, ein Bis- oder Tris-Isocyanat, ein Acyryloyl-Isocyanat, ein vernetzbares Polymer auf der Basis vernetzbarer Silikonhydrogelcopolymere, insbesondere auf der Basis von Co-Polykondensaten aus Bis-amono-demethylsiloxanen und/oder hydrophilen Di- und/oder Triisocyanaten und/oder Acryloyl-Isocyanaten. Wiederum alternativ oder zusätzlich lassen sich auch telechelische Polymere (Telechele) und/oder multivalente hydrophile Polymere einsetzen, also hydrophile Polymere mit vernetzbaren Endgruppen wie beispielsweise Acryl- und/oder Acrylamid-Gruppen. Als hydrophile Ausgangsmonomere kommen beispielsweise ein oder mehrere der folgenden Monomereinheiten in Betracht: Vinylpyrolidon, Hydroxyethylmethacrylat, Hydroxyethylacrylat, Dimethylacrylamid, Acrylamid, Acrylsäure. Telechele und multivalente Polymere können beispielsweise durch die üblichen, lebenden Polymerisationen oder durch Verwendung von funktionellen Kettenübertragungsreagenzien hergestellt werden.

Wie oben dargelegt, ist es besonders bevorzugt, wenn der Formkörper unmittelbar mit dem Körpergewebe in Kontakt steht. Dies bedeutet, dass auf eine Ummantelung des Formkörpers, insbesondere im Sensorbereich und/oder auch im Bereich des Koppelelements, verzichtet werden kann. Sofern dies erforderlich ist, kann der Formkörper jedoch dennoch mit einer Beschichtung versehen werden, insbesondere mit einer biokompatiblen Beschichtung. Insbesondere kann hierfür eine Mehrschichtbeschichtung (beispielsweise nach einem Layer-by-Layer-Verfahren) und/oder eine Plasmabeschichtung eingesetzt werden.

Da der Formkörper in ein Körpergewebe implantiert wird, kann der Formkörper weiterhin mindestens einen heilungsfördernden Wirkstoff umfassen. Dieser heilungsfördernde Wirkstoff kann wiederum in einer Beschichtung um den Formkörper herum und/oder im Formkörper selbst angeordnet sein. Dabei sollte der heilungsfördernde Wirkstoff derart in und/oder auf dem Formkörper angeordnet sein, dass dieser in das umgebende Körpergewebe diffundieren kann, um dort die Heilung zu beschleunigen. Der heilungsfördernde Wirkstoff kann beispielsweise ein Cortison und/oder ein Cortison-Derivat, insbesondere Dexametason, umfassen. Auch andere heilungsfördernde Wirkstoffe sind jedoch selbstverständlich einsetzbar. Der heilungsfördernde Wirkstoff kann insbesondere dafür sorgen, dass sich nach der Implantation schnell oberhalb des Sensorelements eine vollständige geschlossene Gewebeschicht ausbildet, im Gegensatz zu herkömmlichen Sensorelementen, welche in der Regel aus den Gewebeschichten herausragen.

Das beschriebene Sensorelement weist gegenüber dem stand der Technik eine Vielzahl von Vorteilen auf. So ist das Sensorelement so ausgebildet, dass ein Teil des Formkörpers als Sensorbereich ausgebildet ist, wohingegen der andere Teil, das Koppelelement, als transparentes "Fenster" für eine optische Messung eines Sensorsignals dienen kann. Biosensoren, deren analytspezifische Veränderung durch optische Messungen ausgelesen wird, weisen bei Anwendung unter der Haut insbesondere häufig den Nachteil auf, dass die Haut sehr stark im Bereich des sichtbaren Spektrums absorbiert und darüber hinaus eine starke diffuse Streuung aufweist. Absorption und Streuung führen jedoch zu hohen Intensitätsverlusten und zu Artefakten, deren Ursprung in dem sich verändernden Streuverhalten der Haut begründet ist. Beide Effekte erschweren präzise Messungen mit optischen Sensoren unter der Haut. Erschwerend kommt hinzu, dass Sensoren zur Messung von Metaboliten unter der Haut üblicherweise in relativ tiefe Hautschichten, welche gut durchblutet sind, eingesetzt werden müssen. Ein Einsatz in den obersten Hautschichten ist aus diesem Grund jedoch nicht möglich.

Bei dem erfindungsgemäßen Sensorelement hingegen wird ein "Schichtaufbau" verwendet, bei welchem der Sensorbereich in tiefen Hautschichten angeordnet werden kann. Das darüberliegende Koppelteil, welches kein Lichtwellenleiter im klassischen Sinne sein muss, dient dann lediglich noch dazu, ein optisch transparentes "Fenster" hin zur Hautoberfläche oder bis dicht unter die Hautoberfläche zu bilden. Dieser transparente Teil des Koppelteils dient zum unbeeinflussten Weiterleiten des Lichts. Dabei muss dieser Koppelteil nicht notwendigerweise, wie bei einem klassischen Lichtleiter, einen Kern mit hohem Brechnungsindex und eine Hülle mit niedrigerem Brechnungsindex aufweisen, sondern kann ein homogenes, transparentes Material sein. Ein Hülle-Kern-Aufbau ist wegen der geringen Länge des Implantats nicht notwendig.

In dem Sensorbereich werden, zusätzlich zu dem mindestens einen Sensormaterial, weiterhin ein oder mehrere Referenzmaterialien in dem Matrixmaterial eingebettet. Insbesondere kann es sich dabei wiederum um Referenzpartikel handeln, wobei jedoch auch andere Arten von Referenzmaterialien möglich sind, beispielsweise Referenzmoleküle oder Ähnliches. Dieses Referenzmaterial soll derart gewählt sein, dass dieses mindestens eine optisch messbare Eigenschaft aufweist (beispielsweise wiederum eine Lumineszenz - und/oder Fluoreszenzeigenschaft, eine Farbe oder Ähnliches), welche sich auch bei Anwesenheit des mindestens einen Analyten nicht wesentlich ändert. Auf diese Weise können beispielsweise mittels der Messung der optischen Eigenschaft des Referenzmaterials Kalibrationen und/oder Abgleichmessungen durchgeführt werden, um beispielsweise Intensitätsschwankungen einer Lichtquelle und/oder Umgebungseinflüsse der Hautpartien bei der Messung zu eliminieren. Das Referenzmaterial kann beispielsweise in dem Matrixmaterial immobilisiert sein. Das Referenzmaterial kann beispielsweise wiederum in Form von Mikro- oder Nanopartikeln in das Matrixmaterial eingebracht sein, in Form von Mikro- oder Nanokapseln und/oder es kann an das Matrixmaterial chemisch gebunden sein.

Das Sensorelement in einer der oben beschriebenen Ausführungsformen lässt sich insbesondere im Rahmen einer erfindungsgemäßen Sensoranordnung einsetzen. Eine derartige Sensoranordnung umfasst mindestens ein Sensorelement nach einer oder mehreren der oben beschriebenen Ausführungsformen. Weiterhin umfasst die Sensoranordnung mindestens einen optischen Detektor, wobei der Detektor eingerichtet ist, um bei in ein Körpergewebe implantiertem Sensorelement optisch an das Koppelende des Sensorelements anzukoppeln und die mindestens eine optisch messbare Eigenschaft des Sensormaterials zu messen.

Zu diesem Zweck kann der Detektor beispielsweise einen Detektor für elektromagnetische Strahlen umfassen, beispielsweise eine Fotodiode, eine Fotozelle, oder eine andere Art von Detektor für elektromagnetische Strahlen. Weiterhin können ein oder mehrere Vorrichtungen zur spektralen Trennung vorgesehen sein, beispielsweise Gitter, Filter, dichroitische Spiegel oder ähnlich Einrichtungen. Neben einem derartigen Detektor kann die Sensoranordnung weiterhin, je nach Art der nachzuweisenden optischen Eigenschaft des Sensormaterials und/oder des Referenzmaterials, weiterhin auch eine Strahlenquelle zur Erzeugung elektromagnetischer Strahlen und zur Einkopplung dieser Strahlen in das Koppelende des Sensorelements umfassen. Beispielsweise können zu diesem Zweck Leuchtdioden, Glühlampen, Gasentladungslampen oder andere Arten von Lichtquellen vorgesehen sein. Auf diese Weise kann beispielsweise eine Fluoreszenz und/oder Lumineszenz in dem Sensormaterial und/oder dem Referenzmaterial angeregt werden. Wird lediglich eine Farbänderung gemessen, so kann, alternativ oder zusätzlich, auch auf eine Einstrahlung von Anregungslicht verzichtet werden. Weiterhin können für die Messung des Referenzmaterials optional getrennte Detektoren und/oder getrennte Lichtquellen vorgesehen sein, so dass die optische Eigenschaft des Sensormaterials und die optische Eigenschaft des Referenzmaterials getrennt nachgewiesen werden können. Auch eine teilweise oder vollständige Zusammenlegung der für die Messung erforderlichen Komponenten ist jedoch möglich.

Wie oben beschrieben, ist es, im Gegensatz zu den aus dem Stand der Technik bekannten Sensoranordnungen, welche Lichtwellenleiter-Fasern verwenden, bei dem vorgeschlagenen Sensorelement nicht notwendig, das Koppelteil unmittelbar an den optischen Detektor bzw. eine den optischen Detektor und/oder eine oder mehrere Quellen aufnehmende Detektionseinrichtung anzukoppeln. So können der optische Detektor und das Sensorelement bzw. das Koppelende des Sensorelements auch durch mindestens eine Hautschicht bzw. Gewebeschicht voneinander getrennt sein. Das Koppelteil stellt lediglich ein breites "Fenster" zur Verfügung, durch welches der Sensorbereich "beobachtet" werden kann. Eine geringfügige Bedeckung der Oberfläche des Sensorelements durch Gewebe ist - im Gegensatz zu Lichtwellenleiter-Fasern - möglich.

Die Sensoranordnung kann somit derart aufgebaut sein, dass diese räumlich getrennt das Sensorelement und den Detektor bzw. eine den Detektor und gegebenenfalls ein oder mehrere Strahlenquellen umfassende Detektionseinrichtung beinhaltet. Weiterhin können, insbesondere innerhalb der Detektionseinrichtung, zusätzliche Elemente umfasst sein, wie beispielsweise Elemente zum Auswerten der Messung, wie beispielsweise ein oder mehrere Ein- und Ausgabeelemente, ein oder mehrere Datenverarbeitungsgeräte (zum Beispiel Mikroprozessoren), flüchtige und/oder nicht-flüchtige Speicher, oder weitere Elemente. Auch eine oder mehrere Energieversorgungen und/oder Einrichtungen zum Ankoppeln einer externen Energieversorgung an die Detektionseinrichtung können vorgesehen sein.

Neben dem oben beschriebenen Sensorelement und der Sensoranordnung in einer der dargestellten Ausführungsformen wird weiterhin ein Verfahren zur Herstellung eines Sensorelements zur Erfassung mindestens eines Analyten in einer Körperflüssigkeit oder einem Körpergewebe vorgeschlagen. Insbesondere kann das vorgeschlagene Verfahren genutzt werden, um ein Sensorelement nach einer der oben beschriebenen Ausführungsformen herzustellen, so dass für mögliche Definitionen und bevorzugte Ausführungsformen des Sensorelements weitgehend auf die obige Beschreibung verwiesen werden kann.

Bei dem erfindungsgemäßen Verfahren wird eine erste Präpolymerflüssigkeit mit mindestens einem ersten aushärtbaren Präpolymer zur Herstellung eines optisch transparenten Koppelteils in eine Kanüle eingebracht. Unter einer "Präpolymerflüssigkeit" ist dabei eine beliebige Flüssigkeit (d. h. beispielsweise eine Lösung und/oder Emulsion und/oder Suspension) zu verstehen, welche, beispielsweise durch eine chemische Reaktion und/oder eine Phasenänderung, ausgehärtet werden kann. Beispielsweise kann die Präpolymerflüssigkeit den oben beschriebenen mindestens einen Precursor umfassen, wobei die Präpolymerflüssigkeiten verschiedene oder auch identische Precursor umfassen können. Unter "Aushärten" ist dabei eine Änderung von einem flüssigen Zustand in einen festen Zustand zu verstehen, wobei diese Änderung jedoch auch lediglich teilweise vollzogen werden kann, so dass auch nach dem Aushärten noch eine gewisse Verformbarkeit des ausgehärteten Präpolymers bestehen kann. Der Aushärtungsvorgang kann insbesondere initiierbar sein, beispielsweise durch thermische, chemische, photochemische oder andere Arten von Initiierungen.

Weiterhin wird eine zweite Präpolymerflüssigkeit mit mindestens einem zweiten aushärtbaren Präpolymer zur Herstellung eines Sensorbereichs in die Kanüle eingebracht. Dieser Verfahrensschritt kann vor oder nach dem oben beschriebenen Verfahrensschritt des Einbringens der ersten Präpolymerflüssigkeit, oder auch gleichzeitig, erfolgen. Die zweite Präpolymerflüssigkeit kann von der ersten Präpolymerflüssigkeit verschieden sein, kann jedoch auch ganz oder teilweise mit der ersten Präpolymerflüssigkeit identisch sein. In letzterem Fall lassen sich beispielsweise auch die Verfahrensschritte der Einbringung der Präpolymerflüssigkeiten in die Kanüle zu einem einzelnen Verfahrensschritt zusammenfassen. Dann ist allerdings eine getrennte Einbringung des Sensormaterials in die zweite Präpolymerflüssigkeit bzw. den Bereich der Präpolymerflüssigkeit, welcher den Sensorbereich bildet, erforderlich, beispielsweise durch nachträgliche Eindiffusion. In diesem Fall unterscheiden sich die "erste" Präpolymerflüssigkeit und die "zweite" Präpolymerflüssigkeit erst nachträglich durch ihre Funktion, nämlich nachdem das Koppelteil und der Sensorbereich ausgebildet sind.

Weiterhin wird mindestens ein Sensormaterial in die zweite Präpolymerflüssigkeit eingebracht, wobei das Sensormaterial bei Anwesenheit des Analyten mindestens eine optisch messbare Eigenschaft ändert. Das Einbringen des Sensormaterials kann vor oder auch nach dem Einbringen der Präpolymerflüssigkeit in die Kanüle erfolgen. So kann beispielsweise das Sensormaterial vor einem Einpressen und/oder Einsaugen der zweiten Präpolymerflüssigkeit in die Kanüle erfolgen und/oder es kann auch nachträglich, d. h. nachdem sich die zweite Präpolymerflüssigkeit bereits in der Kanüle befindet, ein Einbringen des Sensormaterials in die zweite Präpolymerflüssigkeit erfolgen, beispielsweise durch eine Eindiffusion. Bezüglich der möglichen Ausgestaltungen des Sensormaterials kann weitgehend auf die obige Beschreibung verwiesen werden.

Anschließend werden die erste Präpolymerflüssigkeit und die zweite Präpolymerflüssigkeit vernetzt, so dass ein Formkörper mit einem Sensorende und einem Koppelende entsteht.

Bezüglich der möglichen Ausgestaltungen der Präpolymerflüssigkeit kann weitgehend auf die obige Beschreibung verwiesen werden, so dass insbesondere die oben dargestellten vernetzbaren Kunststoffe bzw. Materialien für das erste Präpolymer bzw. das zweite Präpolymer einsetzbar sind. Besonders bevorzugt ist, wie oben ausgeführt, die Verwendung von Hydrogel, da sich Hydrogel-Formkörper besonders einfach durch aufeinanderfolgendes Aufziehen von Präpolymerlösungen in eine geeignete Kanüle herstellen lassen. Aufgrund der hohen Viskosität und des geringen Kanülenquerschnitts vermischen sich dabei die beiden Präpolymerflüssigkeiten nicht, und in der Kanüle liegen vorzugsweise die Präpolymerlösungen für das Koppelteil und den Sensorbereich schichtweise und getrennt voneinander vor. Die Präpolymerflüssigkeiten können nun entweder in der Kanüle vernetzt werden (beispielsweise photochemisch in einer Glaskanüle und/oder thermisch, beispielsweise in einer Stahlkanüle) und anschließend mit der Kanüle in die Haut injiziert werden. Diese Injektion kann beispielsweise durch ein Einstechen der Kanüle in einen Hautbereich erfolgen, wobei bei einem langsamen Zurückziehen der Kanüle aus dem Hautbereich das Implantat aus der Kanüle herausgedrückt wird. Eine alternative Lösung besteht darin, die Präpolymerflüssigkeiten beim Injizieren unmittelbar auszuhärten, beispielsweise durch Verwendung von UV-Licht. Allgemein kann die Aushärtung bzw. Vernetzung photochemisch, insbesondere durch UV-Licht, thermisch oder auf andere Weise erfolgen.

Wie oben beschrieben, eignen sich als erste und/oder zweite Präpolymerflüssigkeit insbesondere ein Nelficon-Polymer, ein vernetzbares Polyvinylacetat oder ein vernetzbares Derivat des Polyvinylacetats, ein vernetzbarer Polyvinylalkohol oder ein vernetzbares Derivat des Polyvinylalkohols. Beispiele derartiger vernetzbarer PVA-Derivate sind in EP 641 806 B1, EP 790 258 B1 oder in EP 807 265 B1 dargestellt. Auch andere vernetzbare Präpolymere, wie beispielsweise auf Polyethylenglycol-Basis (zum Beispiel Bis-Acryloyl-PEG, Bis-Acrylamido-PEG, Polyurethane aus PEG, Bis- oder Tris-Isocyanate und Acryloyl-Isocyanate) oder auf Basis vernetzbarer Silikonhydrogelcopolymere (Co-Polykondensate aus Bis-Amino-dimethylsiloxanen und hydrophilen Di- oder Tri-Isocyanaten und Acryloyl-Isocyanaten) sind möglich. Auch ist eine Mischung aus verschiedenen Präpolymeren für den transparenten und den Sensor-Teil möglich.

Besonders bevorzugt ist es, wenn das erste aushärtbare Präpolymer und das zweite aushärtbare Präpolymer zumindest teilweise chemisch gleich sind und insbesondere ein gemeinsames aushärtbares Matrixmaterial umfassen. Auf diese Weise lassen sich insbesondere die oben beschriebenen Sensorelemente mit dem gemeinsamen Matrixmaterial für das transparente Koppelteil und den Sensorbereich herstellen. Es sind jedoch auch Ausführungsformen mit unterschiedlichen Präpolymeren bzw. Matrixmaterialien für Koppelteil und Sensorbereich mittels des vorgeschlagenen Verfahrens herstellbar.

Der Formkörper kann insbesondere im ausgehärteten Zustand zumindest teilweise in seiner äußeren Form der Form des Innenlumens der Kanüle entsprechen. Dies kann insbesondere dadurch bewerkstelligt werden, dass die Aushärtung ganz oder teilweise innerhalb der Kanüle erfolgt, beispielsweise innerhalb einer transparenten Kanüle bei einer photochemischen Aushärtung (zum Beispiel einer UV-Bestrahlung) oder einer thermisch leitfähigen Kanüle (zum Beispiel einer Stahlkanüle) bei einer thermischen Initiierung des Aushärtungsvorgangs. Auf diese Weise kann das Aushärten gleichzeitig mit einer Formgebung verbunden werden. Die Vernetzung bzw. Aushärtung kann ganz oder teilweise innerhalb/oder außerhalb der Kanüle erfolgen.

Zum Einbringen der Präpolymerflüssigkeiten in die Kanüle können verschiedene Techniken verwendet werden. So kann insbesondere ein Einsaugen mittels Unterdruck und/oder ein Einpressen mittels Überdruck aus mindestens einem Präpolymervorrat in die Kanüle erfolgen.

Es sei dabei darauf hingeweisen, dass unter einer "Kanüle" im Rahmen der vorliegenden Erfindung ein im Wesentlichen rohrförmiges Gebilde verstanden wird, welches ein Innenlumen aufweist. Dieses Innenlumen kann einen konstanten und/oder einen variablen Querschnitt umfassen. Anstelle eines einzelnen Innenlumens können jedoch auf Kanülen verwendet werden, welche mehrere Innenlumina aufweisen, so dass sich beispielsweise auch mehrschichtige Sensorelemente ausbilden lassen. So kann die Kanüle beispielsweise mehrere ringförmig umeinander angeordnete Lumina umfassen, in welche beispielsweise unterschiedliche Präpolymerflüssigkeiten aufgenommen werden können. Auch benachbarte Anordnungen mehrerer ringförmiger Lumina sind denkbar.

Wie oben beschrieben, ist insbesondere die Verwendung von Hydrogelmaterialien bevorzugt, da derartige Materialien eine besonders gute Biokompatibilität aufweisen und insbesondere auch ohne Beschichtung verwendet werden können. Es ist jedoch auch möglich, zusätzlich eine Beschichtung vorzusehen. Eine derartige Beschichtung kann beispielsweise mindestens eine der folgenden Beschichtungen umfassen:
- eine biokompatible Beschichtung, insbesondere eine Hydrogel-Beschichtung;
- eine Mehrschicht-Beschichtung;
- eine Beschichtung mit mindestens einem heilungsfördernden Wirkstoff;
- eine Beschichtung mit mindestens einem zweiten Sensormaterial, welches bei Anwesenheit des Analyten mindestens eine optisch messbare Eigenschaft ändert.

Derartige Beschichtungen können mittels verschiedener Verfahren aufgebracht werden. Besonders bevorzugt sind hierbei Tauchverfahren, insbesondere Tauchverfahren mit anschließendem Vernetzungsschritt zur Vernetzung der Beschichtung, Coextrusionsverfahren mittels einer Kanüle mit mindestens zwei Extrusionslumina, wobei mindestens ein erstes Extrusionslumina zur Erzeugung der Beschichtung und mindestens ein zweites der Extrusionslumina zur Erzeugung des Koppelteils und/oder des Sensorbereichs verwendet wird. Auch andere Verfahren zur Erzeugung der Beschichtung sind jedoch denkbar, beispielsweise Tauchverfahren in LbL-Technik (Layer-by-Layer) und/oder Plasmabeschichtungen. Vor dem Aufbringen der Beschichtung kann weiterhin mindestens ein Vorbehandlungsschritt zur Verbesserung der Haftung der Beschichtung auf dem Formkörper durchgeführt werden, beispielsweise eine chemische, thermische oder photochemische Vorbehandlung.

Neben dem Sensorelement und dem Verfahren zur Herstellung eines Sensorelements wird weiterhin eine Vorrichtung zur Erzeugung eines Sensorelements vorgeschlagen. Die Vorrichtung kann insbesondere zur Erzeugung eines Sensorelements gemäß einem oder mehrerer der oben beschriebenen Ausführungsbeispiele verwendet werden, so dass wiederum weitgehend auf die obige Beschreibung und die obigen Möglichkeiten der Ausgestaltungen verwiesen werden kann. Die Vorrichtung ist eingerichtet, um ein Verfahren nach einem der vorhergehenden Verfahrensansprüche durchzuführen. Zu diesem Zweck weist die Vorrichtung jeweils Mittel zur Durchführung der einzelnen Verfahrensschritte auf. Dabei können die einzelnen Verfahrensschritte manuell und/oder auch teil- oder vollautomatisch durchgeführt werden.

Die Vorrichtung kann insbesondere eingerichtet sein, um das Sensorelement nicht nur herzustellen, sondern dieses auch in eine Körperflüssigkeit und/oder ein Körpergewebe zu implantieren. Zu diesem Zweck weist die Vorrichtung mindestens eine Kanüle zum Durchdringen eines Hautbereichs eines Patienten auf. Zu diesem Zweck kann die Kanüle beispielsweise mit einem scharfen Ende, einer Spitze, einem Perforationsbereich oder einer ähnlichen Perforationseinrichtung ausgestaltet sein, welche den Hautbereich durchstechen und/oder durchschneiden kann. Alternativ könnte jedoch auch ein separater Schnitt in eine Hautpartie durchgeführt werden, um anschließend die Kanüle einzuführen.

Die Vorrichtung kann weiterhin mindestens eine Vorrichtung zum Einstellen und/oder Begrenzen der Implantationstiefe aufweisen. Beispielsweise kann eine derartige Vorrichtung zur Einstellung und/oder Begrenzung der Implantationstiefe einen Tiefenanschlag umfassen. Mittels einer derartigen Vorrichtung kann stets gewährleistet sein, dass die Implantationstiefe, von deren Gleichmäßigkeit die Qualität der optischen Kopplung wesentlich abhängt, bei allen Implantationen gleichmäßig bzw. reproduzierbar ist.

Weiterhin kann die Vorrichtung mindestens einen Vorratstank zur Aufnahme der ersten Präpolymerflüssigkeit und/oder der zweiten Präpolymerflüssigkeit umfassen. Es können getrennte oder gemeinsame Vorratstanks vorgesehen sein. Weiterhin kann die Vorrichtung mindestens eine Druckvorrichtung zur Erzeugung eines Überdrucks und/oder eines Unterdrucks umfassen, um auf diese Weise ein Einsaugen und/oder Einpressen der ersten und/oder der zweiten Präpolymerflüssigkeit in die Kanüle zu gewährleisten.

Die Vorrichtung kann insbesondere mindestens ein Zulaufventil aufweisen, wobei das Zulaufventil mit einem Hilfsstoffreservoir verbunden ist. Das Hilfsstoffreservoir kann beispielsweise eine Kochsalzlösung oder eine andere Art von fluidem Hilfsstoff sein, welche beispielsweise zum Ausgleich von Unterdrücken innerhalb des Vorratstanks dienen kann.

Die Druckvorrichtung kann beispielsweise mindestens einen bei einer Implantation feststehenden Kolben umfassen, beispielsweise einen mit dem mindestens einen Vorratstank und/oder der mindestens einen Kanüle in Verbindung stehenden feststehenden Kolben. Unter "bei einer Implantation feststehen" wird dabei eine Vorrichtung verstanden, bei welcher zwar die restliche Vorrichtung relativ beispielsweise zu einer Hautoberfläche bewegbar angeordnet ist, der Kolben selbst jedoch (beispielsweise mittels eines geeigneten Anschlags auf der Hautoberfläche) relativ zur Hautoberfläche feststehend positioniert wird. Auf diese Weise kann beispielsweise beim Einführen der Kanüle ein Unterdruck in einem Vorratstank für das mindestens eine Präpolymer erzeugt werden, welcher durch Nachströmen der Hilfsflüssigkeit ausgeglichen wird. Beim erneuten Herausziehen der Kanüle aus dem Hautbereich wird dann der Vorratstank gegen den feststehenden Kolben bewegt, wodurch innerhalb des Vorratstanks ein Überdruck entsteht. Durch diesen Überdruck kann dann das in der Kanüle ausgebildete Implantat in den Hautbereich ausgeschoben werden.

Dieses Prinzip lässt sich allgemein dadurch verallgemeinern, dass die Vorrichtung eingerichtet sein kann, um eine Kanüle in ein Gewebe einzuführen und anschließen wieder aus dem Gewebe zu entfernen, wobei beim Entfernen der Kanüle automatisch ein Sensorelement aus der Kanüle in das Gewebe geschoben wird.

Um insbesondere das Entfernen der Kanüle aus einer Hautpartie zu erleichtern, kann die Vorrichtung weiterhin mindestens ein Rückholfederelement (beispielsweise eine Spiralfeder, eine Blattfeder, ein elastisches Element oder eine andere Art von Federelement) umfassen, welches eingerichtet ist, um nach einer Implantationsbewegung die Vorrichtung wieder ganz oder teilweise aus der Körperflüssigkeit und/oder dem Körpergewebe zu entfernen.

Wie oben beschrieben, kann die Kanüle mit konstantem oder variierendem Querschnitt ausgestaltet sein. Besonders bevorzugt ist es jedoch, wenn die Kanüle in einem vorgegebenen Abstand zur Spitze der Kanüle eine Verengung (beispielsweise eine Einschnürung und/oder eine Verengung, insbesondere eine konische Verengung) aufweist. Eine derartige Verengung kann auf verschiedene Weise vorteilhaft genutzt werden. Zum einen stellt eine derartige Verengung, beispielsweise um bis zu 1 Prozent, bis zu 10 Prozent oder sogar bis zu 20 Prozent oder mehr des Querschnitts der Kanüle, eine "Sollbruchstelle" bei der Bildung des Sensorelements dar, an welcher sich beispielsweise Präpolymerlösung im ausgehärteten oder halb ausgehärteten Zustand von darüberliegender Präpolymerlösung, welche mit einem Vorratstank in Verbindung steht, trennt. An dieser Stelle findet beim Entfernen der Kanüle aus dem Körpergewebe mit hoher Wahrscheinlichkeit ein Abriss statt.

Weiterhin kann die Verengung auch beim Entfernen des Implantats aus dem Gewebe genutzt werden, da über die Kanüle das Sensorelement (und gegebenenfalls umgebende Körperflüssigkeit) angesaugt werden können, solange, bis die sich verjüngende Nadel bzw. Kanüle durch das Implantat verstopft ist. Anschließend kann die Kanüle inklusive dem Sensorelement bzw. Implantat wieder aus der Haut gezogen werden. Somit können dieselbe und/oder ähnliche Vorrichtungen sowohl für die Implantation als auch für das Entfernen des Sensorelements aus der Hautpartie genutzt werden. Der Abstand zwischen der Spitze der Kanüle und der Verengung ist somit vorzugsweise im Wesentlichen gleich der Länge des implantierten Sensorelements.

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: ein in einem Körpergewebe implantiertes erfindungsgemäßes Sensorelement;
- Figuren 2A bis 2F: ein erfindungsgemäßes Verfahren zur Herstellung eines Sensorelements; und
- Figuren 3A bis 3C: eine erfindungsgemäße Vorrichtung zur Herstellung und Implantierung eines Sensorelements.

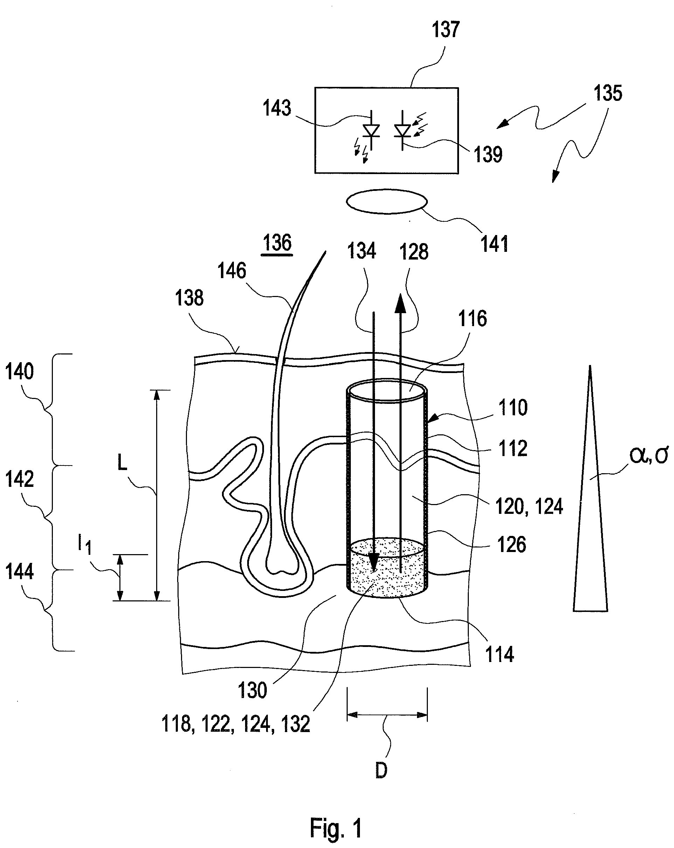

In Figur 1 ist ein erfindungsgemäßes Sensorelement 110 im implantierten Zustand dargestellt. Das Sensorelement 110 weist einen einstückigen Formkörper 112 mit einem Sensorende 114 und einem Koppelende 116 auf. Der Formkörper 112 ist in diesem Ausführungsbeispiel als durchgehender Hydrogel-Formkörper ausgebildet und umfasst beispielsweise die oben beschriebenen Materialien. Der Formkörper 112 weist in diesem Beispiel eine im Wesentlichen zylinderförmige Gestalt auf, mit einem Durchmesser D von ca. 200-500 Mikrometern und einer Gesamtlänge L von ca. 2-5 Millimetern. Dabei ist das Sensorelement 110 unterteilt in einen Sensorbereich 118, welcher im implantierten Zustand ins Innere des Gewebes hineinweist, und ein transparentes Koppelteil 120. Der Sensorbereich 118 weist eine Länge l₁ von ca. 500 Mikrometern auf. Größere Abmessungen sind unter Umständen nachteilig, da in diesem Fall die Ansprechzeiten des Sensorelements 110 auf Grund der langen Diffusionswege zu lang werden. In dem Sensorbereich ist ein Sensormaterial 122 in ein Matrixmaterial 124 eingebettet, wobei das Matrixmaterial 124 auch im Bereich des Koppelteils 120 enthalten ist.

Weiterhin ist dargestellt, dass das Sensorelement 110 optional von einer Beschichtung 126 umgeben sein kann, beispielsweise einer biokompatiblen Beschichtung und/oder einer Beschichtung mit einem heilungsfördernden Wirkstoff. Diese Beschichtung 126 kann beispielsweise in einem LbL-Verfahren (Layer-by-Layer) oder einem Plasmabeschichtungsverfahren aufgebracht werden.

Weiterhin ist dargestellt, dass das transparente Koppelteil 120 als "Fenster" für die Auskopplung eines optischen Signals 128 dient. Dieses optische Signal 128 kann beispielsweise ein aus dem Sensormaterial 122 emittiertes und/oder reflektiertes Licht umfassen, wobei emittiertes Licht beispielsweise in Form von Fluoreszenzlicht und/oder Lumineszenzlicht emittiert werden kann. Dieses optische Signal 128 des Sensormaterials 122 ist sensitiv auf die Anwesenheit eines Analyten in einem das Sensorende 114 umgebenden Körpergewebe 130. Weiterhin kann in dem Sensorbereich 118 neben dem Sensormaterial 122 noch ein Referenzmaterial 132 enthalten sein, welches ebenfalls zu dem optischen Signal 128 beiträgt und eine Referenzkomponente dieses optischen Signals 128 reflektieren bzw. emittieren kann. Weiterhin ist in Figur 1 ein optionaler Anregungsstrahl 134 dargestellt, mittels dessen beispielsweise das Sensormaterial 122 und/oder das Referenzmaterial 132 spezifisch angeregt werden kann. Das Erfordernis der Verwendung eines derartigen Anregungsstrahls 134 ist abhängig von der Art des Sensormaterials 122 und/oder des Referenzmaterials 132 bzw. des verwendeten optischen Nachweismechanismus zum Nachweis des mindestens einen Analyten in dem Körpergewebe 130 und/oder einer Körperflüssigkeit, welche den Sensorbereich 118 umgibt. Das Koppelteil 120 dient vorzugsweise nicht als Lichtwellenleiter, d. h. es werden keine wellenleitenden Eigenschaften von Gebilden mit unterschiedlichen Brechungsindices ausgenutzt, sondern der Brechnungsindex im Bereich des Koppelteils 120 ist vorzugsweise im Wesentlichen homogen. Das Koppelteil 120 wirkt somit lediglich als "Fenster" zur "Betrachtung" des Sensorbereichs 118 vom Außenbereich 136 außerhalb der Hautoberfläche 138.

Dabei ist zu erkennen, dass das Sensorelement 110 vorzugsweise derart in das Körpergewebe 130 implantiert ist, dass dessen Koppelende 116 noch unterhalb der Hautoberfläche 138 angeordnet ist. Die Hautoberfläche 138 oberhalb des Koppelendes 116 ist vorzugsweise im Messbetrieb bereits wieder ausgeheilt.

Als Beispiel eines Körpergewebes 130 ist in dem in Figur 1 dargestellten Ausführungsbeispiel eine Hautpartie mit einer Epidermis 140, einer Dermis 142 und einer Subcutis 144 gezeigt, wobei als Beispiel eines Größenvergleichs ein Haar 146 dargestellt ist. Symbolisch sind in Figur 1 weiterhin die Absorption α und die Streuung σ aufgetragen. Dabei ist zu erkennen, dass die Streuung und die Absorption im Bereich der Hautoberfläche 138 gering sind und mit zunehmender Tiefe im Inneren des Körpergewebes 130 anwachsen. Es sei darauf hingewiesen, dass die dargestellte Hautpartie lediglich beispielhaft für einen Ort der Implantation zu verstehen ist, so dass eine Implantation auch in andere Arten von Körpergewebe 130 erfolgen kann, wie beispielsweise ein Gewebe innerhalb eines Auges oder auch in andere Arten von Körpergewebe.

Weiterhin ist in Figur 1 eine erfindungsgemäße Sensoranordnung 135 dargestellt. Diese Sensoranordnung 135 umfasst neben dem Sensorelement 110 eine Detektionseinrichtung 137 mit mindestens einem optischen Detektor 139. Der optische Detektor 139 ist in Figur 1 lediglich symbolisch dargestellt und hier als Photodiode symbolisiert. Es können jedoch, wie oben ausgeführt, eine Vielzahl optischer Detektoren und/oder zusätzlicher Vorrichtungen, beispielsweise Vorrichtungen zur spektralen Trennung des optischen Signals 128, vorgesehen sein, um das optische Signal 128 des Sensormaterials 122 und/oder des Referenzmaterials 132 zu erfassen. Die Detektionseinrichtung 137 ist dabei in Figur 1 ausgestaltet, um an das Koppelende 116 des Sensorelements 110 anzukoppeln, wobei die Kopplung vorzugsweise durch die obersten Schichten des Körpergewebes 130 hindurch erfolgt. Beispielsweise kann die Detektionseinrichtung 137 zu diesem Zweck auf die Hautoberfläche 138 aufgesetzt werden. Die Detektionseinrichtung 137 ist in Figur 1 optional mit zusätzlichen optischen Vorrichtungen 141 ausgestaltet, welche ebenfalls lediglich symbolisch dargestellt sind, und welche beispielsweise entsprechende Optiken (Linsen, Objektive, Blenden o.ä.) umfassen kann.

Weiterhin umfasst die Detektionseinrichtung 137 in dem in Figur 1 dargestellten Ausführungsbeispiel optional mindestens eine Strahlenquelle 143 zur Erzeugung des optionalen Anregungsstrahls 134. Die Strahlenquelle 143 ist wiederum symbolisch als Leuchtdiode dargestellt, wobei jedoch, wie oben beschrieben, eine Vielzahl anderer Arten von Strahlenquellen umfasst sein können.

Neben der optischen Vorrichtung 141, dem optischen Detektor 139 und der Strahlenquelle 143 kann die Detektionseinrichtung 137 weiterhin zusätzliche Komponenten enthalten, wie beispielsweise Ein- und Ausgabemittel, Energieversorgungen, Datenverarbeitungseinrichtungen o.ä. Es wird bezüglich Beispielen möglicher Ausgestaltungen auf die obige Beschreibung verwiesen.

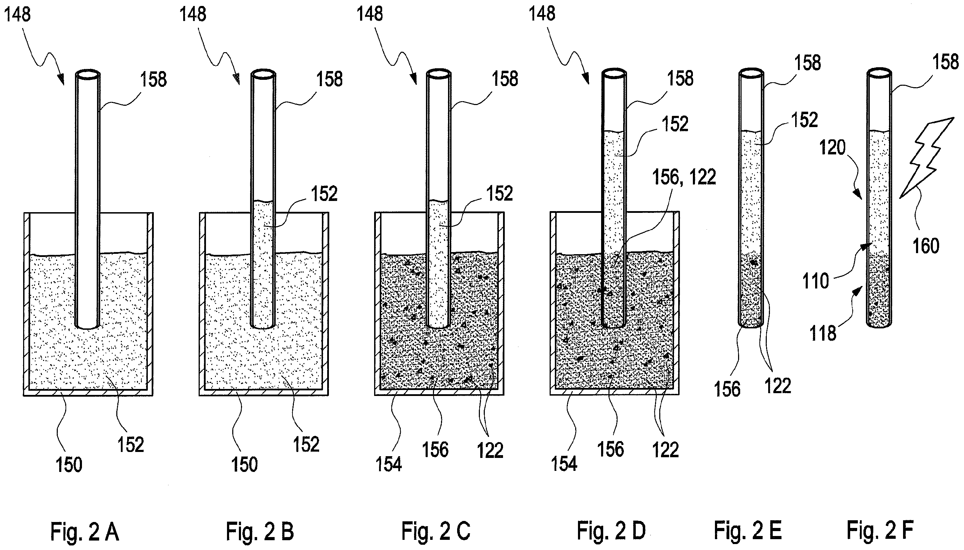

In den Figuren 2A bis 2B sind symbolisch Verfahrensschritte zur Herstellung eines Sensorelements 110, beispielsweise eines Sensorelements 110 gemäß Figur 1, dargestellt. Weiterhin sind in diesen Figuren die bevorzugten Komponenten einer erfindungsgemäßen Vorrichtung 148 eines Sensorelements 110 dargestellt, nämlich ein erster Vorratstank 150 mit einer ersten Präpolymerflüssigkeit 152, ein zweiter Vorratstank 154 mit einer zweiten Präpolymerflüssigkeit 156, mindestens ein Sensormaterial 122, welches in diesem Ausführungsbeispiel in die zweite Präpolymerflüssigkeit 156 eingemischt ist, sowie eine Kanüle 158. Es wird darauf hingewiesen, dass die beiden Vorratstanks 150, 154 auch zusammengefasst sein können, da die beiden Präpolymerflüssigkeiten 152, 156 nicht notwendigerweise getrennt in die Kanüle 158 eingebracht werden müssen, da beispielsweise auch das Einbringen des Sensormaterials 152 nach dem Einbringen der Präpolymerflüssigkeit 152, 156 in die Kanüle 158 erfolgen kann.

Bei der dargestellten Ausführungsform des Verfahrens wird zunächst aus dem ersten Vorratstank 150 eine Schicht der ersten Präpolymerflüssigkeit 152 in die Kanüle 158 eingesaugt (Figuren 2A und 2B). Anschließend wird die Kanüle 158 in den zweiten Vorratstank 154 eingetaucht, und es wird die mit dem Sensormaterial 122 gemischte zweite Präpolymerflüssigkeit 156 als zweite Schicht eingesaugt (Figuren 2C und 2D). Die erste Schicht der ersten Präpolymerflüssigkeit 152 in der Kanüle 158 bildet später den Koppelteil 120 des Sensorelements, wohingegen die zweite Schicht, umfassend die zweite Präpolymerflüssigkeit 156 und das Sensormaterial 122, später den Sensorbereich 118 bildet (siehe Figur 2E).

In Figur 2F ist dargestellt, dass in einem weiteren Verfahrensschritt schließlich die erste Präpolymerflüssigkeit 152 und die zweite Präpolymerflüssigkeit 156 mit dem darin enthaltenen Sensormaterial 122 ausgehärtet wird. Dieses Aushärten kann beispielsweise und vorzugsweise mittels einer Bestrahlung mit UV-Licht 160 erfolgen, wie dies in Figur 2F dargestellt ist. Das Aushärten kann in diesem Fall insbesondere eine photochemische Polymerisation bzw. Vernetzung umfassen. Die erste Präpolymerflüssigkeit 152 und die zweite Präpolymerflüssigkeit 156 werden dabei zu mindestens einem Matrixmaterial 124, wobei bei dem in den Figuren 2A bis 2F dargestellten Verfahren, im Gegensatz zu dem in Figur 1 dargestellten Sensorelement, diese Matrixmaterialien nicht notwendigerweise identisch sein müssen für den Sensorbereich 118 und das Koppelteil 120.

Für das Sensormaterial 122 lassen sich prinzipiell beliebige Sensormaterialien einsetzen, welche auf die Anwesenheit des mindestens einen nachzuweisenden Analyten durch eine Änderung der optischen Eigenschaft reagieren. Aus dem Stand der Technik, beispielsweise dem eingangs beschriebenen Stand der Technik, sind dabei verschiedene Materialien bekannt, die auch im Rahmen der vorliegenden Erfindung eingesetzt werden können. Beispielsweise kann das Sensormaterial 122 Fluorescein-Dextran umfassen, sowie Rhodamin-ConA. Dieses Fluorescein-Dextran bzw. Rhodamin-ConA kann beispielsweise durch Inkubieren in einer wässrigen Lösung in durch ein Verdüsungsverfahren hergestellte Alginatpartikel eingebettet werden. Diese Alginatpartikel können zusätzlich beschichtet werden, beispielsweise durch Mehrfachbeschichtung mit jeweils entgegengesetzt geladenen Polyelektrolytlösungen. Auf diese Weise können die mit dem Sensormaterial beladenen Alginatpartikel mit einer Polyelektrolythülle umgeben werden, welche beispielsweise eine Ausdiffusion des Sensormaterials verhindert. Als Beispiel eines Herstellungsverfahrens, bei welchem derartige Alginatpartikel hergestellt werden, kann beispielsweise auf die WO 2005/079970 A1 verwiesen werden.

Als erste Präpolymerflüssigkeit 152 und/oder als zweite Präpolymerflüssigkeit 156 kann beispielsweise eine Nelficon-Polymerlösung verwendet werden. Für die Vernetzung mit UV-Licht 160 kann beispielsweise eine Quecksilber-Xenon-Lampe verwendet werden, wobei in diesem Fall die Kanüle 158 vorzugsweise als transparente Kanüle 158 ausgebildet ist.

Der in Figur 2F dargestellte Aushärtungs- bzw. Vernetzungsprozess, welcher in diesem Fall durch UV-Licht 160 initiiert wird, kann in verschiedenen Zuständen erfolgen. Zum einen kann dieser Aushärtungsprozess, wie in Figur 2F dargestellt, außerhalb eines Körpergewebes 130 erfolgen, indem beispielsweise die in diesem Fall transparente Kanüle 158 mittels einer UV-Lampe, welche ebenfalls Bestandteil der Vorrichtung 148 sein kann, beleuchtet wird. Alternativ oder zusätzlich kann die Vernetzung bzw. Aushärtung auch innerhalb des Körpergewebes 130 erfolgen, beispielsweise indem die Kanüle 158 in das Körpergewebe 130 eingeführt wird. Für dieses Einführen kann die Hautoberfläche 138 mit einem Schnitt versehen werden, oder die Kanüle 158 selbst kann mit einem scharfen bzw. spitzen Ende ausgestattet werden, mittels dessen die Hautoberfläche 138 perforiert werden kann. Die Beleuchtung mit UV-Licht kann dann in oberen Gewebeschichten des Körpergewebes 130 erfolgen, in welchen die Absorption noch keine allzu großen Werte annimmt, so dass das UV-Licht das Körpergewebe noch durchdringt. Auf diese Weise ist eine besonders hohe Sterilität des Sensorelements 110 gewährleistet, da dieses quasi unmittelbar im Körpergewebe 130 erzeugt wird. Eine dritte Möglichkeit, welche teilweise auch mit den anderen Möglichkeiten kombinierbar ist, besteht darin, das Sensorelement 110 außerhalb der Kanüle 158 und außerhalb des Körpergewebes 130 zu vernetzen bzw. auszuhärten und anschließend zu implantieren.

Die dargestellte Vorrichtung 148 zur Herstellung des Sensorelements 110 kann somit, neben den Vorratstanks 150, 154 und den Präpolymerflüssigkeiten 152, 156 und der Kanüle 158, wie oben beschrieben, weiterhin eine UV-Lichtquelle umfassen, welche in Figur 2F nicht dargestellt ist.

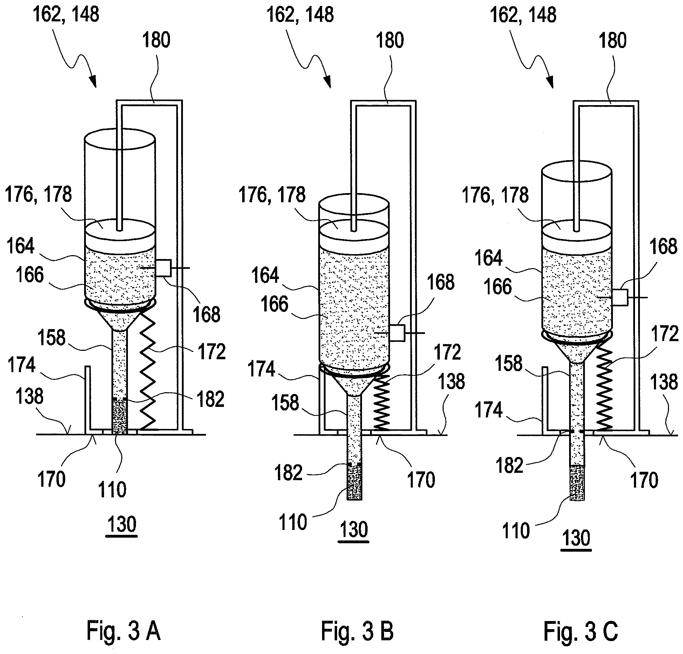

In den Figuren 3A-3C ist eine Vorrichtung 162 zur Implantation eines Sensorelements 110 dargestellt, welche auch gleichzeitig als Vorrichtung 148 zur Herstellung eines Sensorelements genutzt werden kann. Die Vorrichtung 162 umfasst wiederum eine Kanüle 158 zur Perforation einer Hautoberfläche 138. Die Vorrichtung 162 ist dabei in einem Zustand dargestellt, in welchem bereits ein fertiges Sensorelement 110 in der Kanüle 158 angeordnet ist. Dieses Sensorelement 110 kann insbesondere nach dem in den Figuren 2A-2F dargestellten Verfahren innerhalb der Kanüle 158 erzeugt sein. Die Kanüle 158 wirkt in diesem Fall auch als Implantationsnadel.

Oberhalb der Kanüle 158 ist ein Vorratstank 164 angeordnet. Der Vorratstank 164 kann beispielsweise mit Präpolymerflüssigkeit 152, 156 gefüllt sein, kann jedoch auch, nach Herstellung des Sensorelements 110, alternativ oder zusätzlich zur Präpolymerflüssigkeit 152, 156 mit einer Hilfsflüssigkeit 166 gefüllt sein, beispielsweise einer Kochsalzlösung. Diese Hilfsflüssigkeit 166 kann beispielsweise über ein Zulaufventil 168 zugeführt werden, beispielsweise aus einem Hilfsstoffreservoir (in den Figuren nicht dargestellt).

Die Vorrichtung 162 verfügt in dem in den Figuren 3A-3C dargestellten Ausführungsbeispiel über eine (beispielsweise ringförmig um die Kanüle 158 angeordnete) breitflächige Hautauflagefläche 170. Diese Hautauflagefläche 170 wird auf die Hautoberfläche 138 aufgesetzt. Der Vorratstank 162 und die Kanüle 158 werden relativ zu dieser Hautauflagefläche 170 in die Hautoberfläche 138 eingeführt (vgl. Figur 3B). Dabei wird ein Federelement 172 zusammengepresst (vgl. Figur 3B). Über einen Stopper 174 wird die Eindringtiefe und somit die Implantationstiefe eingestellt. Dieser Stopper kann beispielsweise als Tiefenanschlag ausgebildet sein und bildet somit eine Vorrichtung zum Einstellen der Implantationstiefe.

Die Vorrichtung 162 umfasst weiterhin eine Druckvorrichtung 176 in Form eines Kolbens 178. Der Kolben 178 ist in diesem Ausführungsbeispiel innerhalb des Vorratstanks 164 angeordnet, könnte jedoch auch beispielsweise mit der Kanüle 158 ummittelbar verbunden sein. Der Kolben 178 ist derart ausgestaltet, dass dieser bei der Implantation feststeht, d.h. seine Position relativ zur Hautoberfläche 138 nicht verändert. Dies kann beispielsweise, wie in den Figuren 3A-3C dargestellt, mittels eines Gestänges 180 erfolgen, bzw. einer anders gestalteten Vorrichtung, welche den Abstand zwischen der Hautauflagefläche 170 und dem Kolben 178 konstant hält.

Beim Absenken der Kanüle 158 bzw. des Vorratstanks 164 entsteht wegen des feststehenden Kolbens 178 im Inneren des Vorratstanks 164 ein geringer Unterdruck. Über das Zulaufventil 168, welches beispielsweise als Rückschlagventil ausgebildet sein kann, strömt die Hilfsflüssigkeit 166, bevorzugt physiologische Kochsalzlösung, in das Innere des in diesem Ausführungsbeispiel hülsenförmig ausgebildeten Vorratstanks 164.

Der Stopper 174 beendet die Abwärtsbewegung. Durch die Abwärtsbewegung wird das Federelement 172 gespannt, wodurch die Kanüle 158 und der Vorratstank 164 wieder nach oben, d.h. aus der Hauptoberfläche 138 heraus, gedrückt werden. Dann nun das Rückschlagventil des Zulaufventils 168 geschlossen bleibt, entsteht ein Überdruck innerhalb des Vorratstanks 164, welcher das Sensorelement 110 aus der Spitze der Kanüle 158 heraus in das Körpergewebe 130 drückt. Dabei bewegt sich das Sensorelement 110 relativ zur Hautoberfläche 138 nicht mehr.

Beim Absenken des sich aus der Kanüle 158 und dem Vorratstank 164 zusammensetzenden Injektors ist zu berücksichtigen, dass die Hilfsflüssigkeit 166 durch das Rückschlagventil des Zulaufventils 168 leichter einströmen kann als sich das Sensorelement 110 innerhalb der Kanüle 158 bewegen kann. Entsprechend ist es vorteilhaft, wenn die Kanüle 158 sich nach oben hin, d.h. hin zum Vorratstank 164, verjüngt, oder eine Verengung 182 (beispielsweise einen Überstand, eine Verjüngung, einen Vorsprung, eine Sicke o.ä.) nach innen aufweist, was in beiden Fällen dazu führt, dass sich das Sensorelement nicht nach oben in der Kanüle 158 bewegen kann.

Die in den Figuren 3A-3C dargestellte Vorrichtung 162 kann auch zum Entfernen des Sensorelements 110 verwendet werden. Dazu wird die leere, sich nach oben beispielsweise verjüngende oder mit einer Verengung 182 versehene Kanüle 158 über das implantierte Sensorelement injiziert. Durch den durch den Hub des Kolbens 178 erzeugen Unterdruck wird Gewebe und Implantat angesaugt (wobei beispielsweise das Ventil 168 verschlossen werden kann, um den Unterdruck aufrecht zu erhalten), bis sich die verjüngende Kanüle 158 durch das Sensorelement 110 verschließt. Anschließend kann die Kanüle 158 inklusive dem Sensorelement 110 wieder aus der Hautoberfläche 138 herausgezogen werden.

### Bezugszeichenliste

- 110: Sensorelement
- 112: Formkörper
- 114: Sensorende
- 116: Koppelende
- 118: Sensorbereich
- 120: Koppelteil
- 122: Sensormaterial
- 124: Matrixmaterial
- 126: Beschichtung
- 128: optisches Signal
- 130: Körpergewebe
- 132: Referenzmaterial
- 134: Anregungsstrahl
- 135: Sensoranordnung
- 136: Außenbereich
- 137: Detektionseinrichtung
- 138: Hautoberfläche
- 139: Optischer Detektor
- 140: Epidermis
- 141: optische Vorrichtung
- 142: Dermis
- 143: Strahlenquelle
- 144: Subcutis
- 146: Haare
- 148: Vorrichtung zur Herstellung eines Sensorelements
- 150: erster Vorratstank
- 152: erste Präpolymerflüssigkeit
- 154: zweiter Vorratstank
- 156: zweite Präpolymerflüssigkeit
- 158: Kanüle
- 160: UV-Licht
- 162: Vorrichtung zur Implantation eines Sensorelements
- 164: Vorratstank
- 166: Hilfsflüssigkeit
- 168: Zulaufventil
- 170: Hautauflagefläche
- 172: Federelement
- 174: Stopper
- 176: Druckvorrichtung
- 178: Kolben
- 180: Gestänge
- 182: Verengung

## Patentansprüche

1. Sensorelement (110) zur Erfassung mindestens eines Analyten in einer Körperflüssigkeit oder einem Körpergewebe (130), insbesondere zur Bestimmung mindestens einer Metabolitkonzentration in einer Körperflüssigkeit, wobei das Sensorelement (110) einen implantierbaren, einstückigen Formkörper (112) umfasst, wobei der Formkörper (112) ein Sensorende (114) und ein Koppelende (116) umfasst, wobei der Formkörper (112) im Bereich des Sensorendes (114) mindestens einen Sensorbereich (118) umfasst, wobei der Sensorbereich (118) mindestens ein Sensormaterial (122) umfasst, welches bei Anwesenheit des Analyten mindestens eine optisch messbare Eigenschaft ändert, wobei der Formkörper (112) weiterhin mindestens ein optisch transparentes Koppelteil (120) aufweist, wobei das Koppelteil (120) eingerichtet ist, um elektromagnetische Strahlung in mindestens einem Spektralbereich zwischen dem Sensorbereich (118) und dem Koppelende (116) zu transmittieren, wobei der Formkörper (112) im Sensorbereich (118) mindestens ein optisch transparentes Matrixmaterial (124) aufweist, wobei der Analyt zumindest teilweise durch das Matrixmaterial (124) zu dem Sensormaterial (122) diffundieren kann, wobei das Sensormaterial (122) in dem Matrixmaterial (124) eingebettet ist, wobei das Koppelteil (120) zumindest teilweise durch das Matrixmaterial (124) gebildet wird, wobei das Matrixmaterial (124) mindestens einen aushärtbaren, insbesondere einen vemetzbaren, Kunststoff, insbesondere einen biokompatiblen vernetzbaren Kunststoff, in ausgehärteter Form umfasst, **dadurch gekennzeichnet, dass** das Sensormaterial (122) zumindest teilweise in dem Matrixmaterial (124) in Mikrokapseln eingebettet ist, das Koppelteil im Wesentlichen frei von Sensormaterial ist, und in dem Sensorbereich weiterhin mindestens ein Referenzmaterial, insbesondere Referenzpartikel, eingebettet ist, wobei das Referenzmaterial mindestens eine optisch messbare Referenzeigenschaft aufweist, welche sich bei Anwesenheit des Analyten nicht wesentlich ändert.

2. Sensorelement (110) nach einem der vorhergehenden Ansprüche, wobei das Sensormaterial (122) in dem Matrixmaterial (124) immobilisiert ist.

3. Sensorelement (110) nach einem der vorhergehenden Ansprüche, wobei das Matrixmaterial (124) in dem Sensorbereich (118) im implantierten Zustand des Sensorelements (110) unmittelbar mit der Körperflüssigkeit und/oder dem Körpergewebe (130) in Kontakt steht.

4. Sensorelement (110) nach einem der vorhergehenden Ansprüche, wobei das Koppelteil (120) als langgestrecktes Koppelteil (120) mit im Wesentlichen homogenem Brechungsindex ausgebildet ist.

5. Sensorelement (110) nach einem der vorhergehenden Ansprüche, wobei der vemetzbare Kunststoff mindestens ein Hydrogel und/oder mindestens ein statistisches Copolymer umfasst.

6. Sensorelement (110) nach einem der vorhergehenden Ansprüche, wobei der vemetzbare Kunststoff hergestellt ist unter Verwendung mindestens eines der folgender Materialien: ein Nelfilcon-Polymer; ein vernetzbares Polyvinylacetat oder ein vernetzbares Derivat des Polyvinylacetats; ein vernetzbarer Polyvinylalkohol oder ein vernetzbares Derivat des Polyvinylalkohols; ein vernetzbares Polymer auf Polyethylenglycol-Basis, insbesondere auf Basis mindestens eines der folgenden Polymere: Bis Acrylol-Polyethylenglycol, Bis Acrylamido-Polyethylenglycol, ein Polyurethan auf der Basis von Polyethylenglycol, ein Bis- oder Tris-Isocyanat, ein Acyryloyl-Isocyanat; ein vernetzbares Polymer auf der Basis vernetzbarer Silikonhydrogelcopolymere, insbesondere auf der Basis von Co-Polykondensaten aus Bis-amono-demethylsiloxanen und/oder hydrophilen Di- und/oder Triisocyanaten und/oder Acryloyl-Isocyanaten; ein telechelisches Polymer und/oder ein multivalentes hydrophiles Polymer.

7. Sensorelement (110) nach einem der vorhergehenden Ansprüche, wobei der Formkörper (112) ganz oder teilweise mit einer biokompatiblen Beschichtung (126) versehen ist, insbesondere einer Mehrschichtbeschichtung und/oder einer Plasmabeschichtung.

8. Sensorelement (110) nach einem der vorhergehenden Ansprüche, wobei der Formkörper (112) weiterhin mindestens einen heilungsfördernden Wirkstoff umfasst.

9. Sensorelement (110) nach dem vorhergehenden Anspruch, wobei der heilungsfördernde Wirkstoff derart in und/oder auf dem Formkörper (112) angeordnet ist, dass dieser in das umgebende Körpergewebe (130) diffundieren kann.

10. Sensoranordnung (135), umfassend mindestens ein Sensorelement (110) nach einem der vorhergehenden Ansprüche und mindestens einen optischen Detektor (139), wobei der Detektor (139) eingerichtet ist, um bei in ein Körpergewebe (130) implantiertem Sensorelement (110) optisch an das Koppelende (116) anzukoppeln und die mindestens eine optisch messbare Eigenschaft des Sensormaterials (122) zu messen.

## Claims

1. Sensor element (110) for detecting at least one analyte in a body fluid or in a body tissue (130), particularly for determining at least one metabolite concentration in a body fluid, wherein the sensor element (110) comprises an implantable, one-piece shaped body (112), wherein the shaped body (112) comprises a sensor end (114) and a coupling end (116), wherein the shaped body (112) comprises, in the area of the sensor end (114), at least one sensor area (118), wherein the sensor area (118) comprises at least one sensor material (122) which changes at least one optically measurable property in the presence of the analyte, wherein the shaped body (112) also has at least one optically transparent coupling part (120), wherein the coupling part (120) is designed to transmit electromagnetic radiation in at least one spectral range between the sensor area (118) and the coupling end (116), wherein the shaped body (112) has, in the sensor area (118), at least one optically transparent matrix material (124), wherein the analyte can at least partially diffuse through the matrix material (124) to the sensor material (122), wherein the sensor material (122) is embedded in the matrix material (124), wherein the coupling part (120) is formed at least partially by the matrix material (124), wherein the matrix material (124) comprises at least one curable plastic, in particular a cross-linkable plastic, in particular a biocompatible cross-linkable plastic, in cured form, **characterized in that** the sensor material (122) is at least partially embedded in the matrix material (124) in microcapsules, that the coupling part is substantially free of sensor material, and that at least one reference material, particularly in the form of reference particles, is also embedded in the sensor area, wherein the reference material has at least one optically measurable reference property that does not substantially change in the presence of the analyte.

2. Sensor element (110) according to the preceding claim, wherein the sensor material (122) is immobilized in the matrix material (124).

3. Sensor element (110) according to one of the preceding claims, wherein the matrix material (124) in the sensor area (118) is, in the implanted state of the sensor element (110), in direct contact with the body fluid and/or the body tissue (130).

4. Sensor element (110) according to one of the preceding claims, wherein the coupling part (120) is designed as an elongate coupling part (120) with a substantially homogeneous refractive index.

5. Sensor element (110) according to one of the preceding claims, wherein the cross-linkable plastic comprises at least one hydrogel and/or at least one random copolymer.

6. Sensor element (110) according to one of the preceding claims, wherein the cross-linkable plastic is produced by using at least one of the following materials: a nelfilcon polymer; a crosslinkable polyvinyl acetate or a cross-linkable derivative of the polyvinyl acetate; a cross-linkable polyvinyl alcohol or a cross-linkable derivative of the polyvinyl alcohol; a cross-linkable polymer based on polyethylene glycol, in particular based on at least one of the following polymers: bis(acryloyl) polyethylene glycol, bis(acrylamido) polyethylene glycol, a polyurethane based on polyethylene glycol, a bis- or tris-isocyanate, an acryloyl isocyanate; a cross-linkable polymer based on cross-linkable silicone hydrogel copolymers, in particular based on co-polycondenates of bis(aminodimethyl) siloxanes and/or hydrophilic di- and/or tri-isocyanates and/or acryloyl isocyanates; a telechelic polymer and/or a multivalent hydrophilic polymer.

7. Sensor element (110) according to one of the preceding claims, wherein the shaped body (112) is provided completely or partially with a biocompatible coating (126), in particular a multi-layer coating and/or a plasma coating.

8. Sensor element (110) according to one of the preceding claims, wherein the shaped body (112) also comprises at least one active substance that promotes healing.

9. Sensor element (110) according to the preceding claim, wherein the active substance that promotes healing is arranged in and/or on the shaped body (112) in such a way as to be able to diffuse into the surrounding body tissue (130).

10. Sensor arrangement (135) comprising at least one sensor element (110) according to one of the preceding claims and at least one optical detector (139), wherein the detector (139) is designed for optically coupling to the coupling end (116), when the sensor element (110) is implanted in a body tissue (130), and for measuring the at least one optically measurable property of the sensor material (122).

## Revendications

1. Élément capteur (110) destiné à détecter au moins un analyte dans un fluide corporel ou un tissu corporel (130), notamment en vue de déterminer au moins une concentration de métabolite dans un fluide corporel, l'élément capteur comportant un corps façonné (112) monobloc implantable, le corps façonné (112) comportant une extrémité de capteur (114) et une extrémité de couplage (116), le corps façonné (112) comportant au moins une zone de détection dans la zone de l'extrémité de capteur (114), la zone de détection (118) comportant au moins un matériau de capteur (122) qui, en présence de l'analyte, modifie au moins une propriété mesurable de manière optique, le corps façonné (112) possédant en outre au moins une partie de couplage (120) optiquement transparente, la partie de couplage (120) étant conçue pour transmettre un rayonnement électromagnétique dans au moins une plage spectrale entre la zone de détection (118) et l'extrémité de couplage (116), le corps façonné (112) possédant dans la zone de détection (118) au moins un matériau de matrice (124) optiquement transparent, l'analyte pouvant se diffuser au moins partiellement à travers le matériau de matrice (124) vers le matériau de capteur (122), le matériau de capteur (122) étant enrobé dans le matériau de matrice (124), la partie de couplage (120) étant au moins partiellement formée par le matériau de matrice (124), le matériau de matrice (124) comportant au moins une matière plastique durcissable, notamment réticulable, en particulier une matière plastique réticulable biocompatible, sous forme durcie, **caractérisé en ce que** le matériau de capteur (122) est au moins partiellement enrobé dans le matériau de matrice (124) dans des microcapsules, que la partie de couplage est sensiblement exempte de matériau de capteur et au moins un matériau de référence, notamment des particules de référence, étant en outre enrobé dans la zone de détection, que le matériau de référence possédant au moins une propriété de référence mesurable de manière optique qui ne varie pas considérablement en présence de l'analyte.

2. Élément capteur (110) selon la revendication précédente, le matériau de capteur (122) étant immobilisé dans le matériau de matrice (124).

3. Élément capteur (110) selon l'une des revendications précédentes, le matériau de matrice (124), à l'état implanté de l'élément capteur (110), se trouvant directement en contact avec le fluide corporel et/ou le tissu corporel (130) dans la zone de détection (118) .

4. Élément capteur (110) selon l'une des revendications précédentes, la partie de couplage (120) étant réalisé sous la forme d'une partie de couplage (120) allongée avec un indice de réfraction sensiblement homogène.

5. Élément capteur (110) selon l'une des revendications précédentes, la matière plastique réticulable comprenant au moins un hydrogel et/ou au moins un copolymère statistique.

6. Élément capteur (110) selon l'une des revendications précédentes, la matière plastique réticulable étant fabriquée en utilisant au moins l'un des matériaux suivants : un polymère nelfilcon ; un polyacétate de vinyle réticulable ou un dérivé réticulable du polyacétate de vinyle ; un alcool polyvinylique réticulable ou un dérivé réticulable de l'alcool polyvinylique ; un polymère réticulable à base de polyéthylène glycol, notamment à base d'au moins l'un des polymères suivants : bi-acryloyl-polyéthylène glycol, bi-acrylamido-polyéthylène glycol, un polyuréthane à base de polyéthylène glycol, un bi- ou tri-isocyanate, un acyryloyl-isocyanate ; un polymère réticulable à base de co-polycondensats en bi-amono-déméthylsolixane et/ou de bi- et/ou tri-isocyanates et/ou acyryloyl-isocyanate hydrophiles ; un polymère téléchélique et/ou un polymère hydrophile multivalent.

7. Élément capteur (110) selon l'une des revendications précédentes, le corps façonné (112) étant entièrement ou partiellement pourvu d'un revêtement (126) biocompatible, notamment d'un revêtement multicouche et/ou d'un revêtement au plasma.

8. Élément capteur (110) selon l'une des revendications précédentes, le corps façonné (112) comportant en outre au moins une substance active favorisant la guérison.

9. Élément capteur (110) selon la revendication précédente, la substance active favorisant la guérison étant disposée dans et/ou sur le corps façonné (112) de telle sorte que celle-ci peut se diffuser dans le tissu corporel (130) environnant.

10. Arrangement capteur (135), comprenant au moins un élément capteur (110) selon l'une des revendications précédentes et au moins un détecteur optique (139), le détecteur (139) étant conçu pour s'accoupler optiquement à l'extrémité de couplage (116) lorsque l'élément capteur (110) est implanté dans un tissu corporel (130) et mesurer l'au moins une propriété mesurable de manière optique du matériau de capteur (122) .
